# EUROPEAN PATENT APPLICATION

(11) **EP 2 821 789 A1**
(43) Date of publication of application: **07.01.2015**
(21) Application number: 13003337.6
(22) Date of filing: 02.07.2013
(51) Int. Cl.: G01N 33/50

(54) **Microtissues**

(71) Applicant: ETH Zurich, 8092 Zurich (CH)
(72) Inventor: Millan, Christopher, CH - 8002 Zürich (CH); Miranda-Nieves, David, Cambridge, MA 02139 (US); Zenobi-Wong, Marcy, CH - 8049 Zürich (CH); Yang, Yuan, 06110 Halle / Saale (DE); Groth, Thomas, D - 10439 Berlin (DE)

(57) **Abstract**

A method of making a three-dimensional microtissue comprising the reaction of at least two cytocompatible polymer solutions, each polymer comprising complementary reactive groups capable of spontaneous reaction, at least one of which solutions additionally contains cells.

The method allows the rapid, efficient production of microtissues useful for purposes such as pharmaceutical screening and tissue reconstruction.

## Description

This disclosure relates to microtissues adapted for a variety of uses, including forming the basis of pharmaceutical screens.

It has been proposed to make microtissues for use, for example, as pharmaceutical screens using fluorescent detectors, for example, reagent-based fluorescent assay or cells genetically modified to express a fluorescent promoter reporter. Traditionally, these have been two-dimensional surfaces. It is recognised that three-dimensional surfaces would be better in this regard (see, for example, Kisaalita WS. "3D cell-based biosensors in drug discovery programs: Microtissue engineering for high throughput screening": CRC Press; 2010.). Such 3-D structures also have the potential to create building blocks for use in tissue engineering (see, for example, Elbert DL. "Bottom-up tissue engineering. Current opinion in biotechnology". 2011;22:674-80 and Fennema E, Rivron N, Rouwkema J, van Blitterswijk C, de Boer J. "Spheroid culture as a tool for creating 3D complex tissues". Trends in biotechnology. 2013.)

The methods currently available to the art for the creation of such include centrifugation, hanging drop culture, gyratory shakers or spinner flasks. All of these methods, however, depend on the formation of cell-cell and cell-matrix adhesions based on receptors of the cadherin, ICAM and integrin families and thus require culture times on the order of hours, sometimes days, before a robust microtissue is formed. If the cell adhesion receptors are missing on the cell surface, as is often the case of cells which have been cryopreserved, the formation of the spheroid fails.

It has now been found that it is possible to make a desirable 3-D microtissue in as little as a matter of a few minutes. There is therefore provided a method of making a three-dimensional microtissue comprising the reaction of at least two cytocompatible polymer solutions, each polymer comprising complementary reactive groups capable of spontaneous reaction, at least one of which solutions additionally contains cells.

There is additionally provided a three-dimensional microtissue comprising a crosslinked composition of at least two cytocompatible polymers and cells, the crosslinking being provided by complementary reactive groups of two types, one of these types being present on each one of the cytocompatible polymers.

The cytocompatible polymers for use in this method may be any suitable polymers with the necessary cytocompatibility, that is, their presence is not harmful to cells. They may be natural or synthetic materials. The necessary complementary reactive groups may be already present on the polymers, or the polymers may be modified to include such groups. This is within the skill of the art in every case.

Typical non-limiting examples of natural polymers include alginate, alginate sulfate, chondroitin sulfate, dermatin sulfate, hyaluronic acid, cellulose, dextran, poly-1-lysine, chitosan, gelatin, silk and collagen.

Typical non-limiting examples of synthetic polymers include polymers, or polymers derived from, poly ethylene glycol, poly propylene glycol, polaxomers, poly oxazolines, poly ethylenimine, poly vinyl alcohol, poly vinyl acetate, poly methyl vinyl ether-co-maleic anhydride, poly lactide, poly N-isopropylacrylamide, poly glycolic acid, poly methylmethacrylate, poly acrylamide, poly acrylic acid, polyallylamine.

The complementary reactive groups are those that will react spontaneously on being brought together. Many such reactive pairs are known to the art. A typical complementary pair of reactive groups is the aldehyde/amine pairing that can lead to a Schiff base linkage. For example, aldehyde groups may be generated on biopolymers such as alginate, alginate sulfate, chondroitin sulfate, dermatin sulfate, hyaluronic acid, cellulose and dextran by chemical oxidation with suitable reagents, such as sodium periodate, sodium (meta) periodate and hydrogen peroxide. The equivalent amine-containing polymers may include proteins and peptides with a high percentage of lysine like chitosan, gelatin, silk, and collagen and synthetic polymers containing an amine group in their main or side chains like polyallylamine and polyethylenimine.

Another possible complementary reaction may be thiol Michael addition reactions. In this case, thiol groups, which may be naturally present, for example, in amino acids such as cysteine, or introduced chemically may be reacted with Michael acceptors including acrylate esters, acrylonitrile, acrylamides, maleimides, alkyl methacrylates, cyanoacrylates and vinyl sulfones.

In terms of the reactive groups, it is preferred to have a stoichiometry of as close to 1:1 as possible. However, this is not narrowly critical, and a variation of up to 20% is tolerable. In the case of the aldehyde-amine reaction, it is preferred to have an excess of amines, as this is more tolerated by the cells.

The cells which may be present in one or both polymer solutions may be selected from any suitable cells, depending on the desired end use of the microtissue. For example, if a screen is desired, the cells may be genetically modified, so that they can fluoresce in the presence of compound hits. Fluorescence-based assays can be used to detect changes in proliferation, morphology, oxidative stress, cell signally, inflammation, cytotoxicity, genotoxicity. Detection can be performed on a plate reader, confocal microscope, two-photon microscopy to attain information on fluorescent intensity, distribution, polarization and variance with time. Preferred cells types are EGFP reporter cell lines.

There is therefore also provided a screen assay, comprising a microtissue as hereinabove described, in which the cells are adapted to fluoresce on exposure to a stimulus whose detection is desired.

The microtissues of this disclosure are prepared by first providing the cytocompatible polymers with the necessary complementary reactive groups, should they require modification, adding the desired cells to one or both of the solutions and then combining them. Many of the crosslinking reactions occur spontaneously, such that a microtissue is formed and can be handled with forceps after only a few minutes. The microtissues may be made in sheets, pellets or spheroids. They can be made directly in the wells of screening plates.

The size of the microtissue will be determined by the number of starting cells. The number of cells used generally ranges from about 1000 to about 1 million. The quantity of polymer to be used with the chosen quantity of cells is determined by the ability to provide a microtissue that is sufficiently cohesive to be manipulated, for example, picked up with forceps. The quantities of polymer needed will vary in each individual case, depending on polymer type and desired degree of cohesion in a given application, but this can be readily determined in each case by routine, non-inventive experimentation.

The method allows considerable versatility. For example,

The possible uses of these microtissues include, but are not limited to
- Pharmaceutical screens
- Building blocks in tissue engineering.

The method is further described with reference to the following non-limiting examples, which depict particular embodiments.

The figures are as follows:
Figure 1 shows micrographs of the alcian staining of various microtissues. The size bars represent 200 microns (see Example 1 for details)
Figure 2A shows micrographs of a microtissue which has been immunohistochemically stained to detect collagen 2 protein using the monoclonal antibody II-II6B3 from the Development Studies Hybridoma Bank. Figure 2B is a control without any primary antibody. The size bars represent 200 microns
Figure 3 is a graph displaying glycosaminoglycan contents of pellets in chondrogenic media (C), non-chondrogenic media (NC), and microtissues prepared according to Example 1 in chondrogenic media (QS C).
Figure 4 is a micrograph of bovine chondrocytes which have been transduced with a collagen 2 promoter - GFP construct. The cells are suspended in 2% agarose for 7 days and the collagen 2 reporter appears as green in several cells.

### Example 1:

To form microtissues using the Schiff linkage technique, human mesenchymal stem cells (hMSCS, Lonza Group Ltd, Basel, Switzerland) between p.6-p.9, human chondrocytes and human adipose derived stromal cells were suspended in a solution of 5 mg/mL sChi at a cell density of 20 x 10⁶ cells/mL. Drops of 5 µL each of oxidized alginate were prepared on plastic ring structures to confine the drop and 10 µL of the cells + S-Chi were pipetted into individual droplets of oxAlginate. The substrate was turned upside down and the reaction was carried out in an incubator at 37°C for 10 minutes. Microtissues were transferred to agarose coated-wells of a 96-well plate with fine-tipped forceps and cultured in chondrogenic media and growth factor free media. In parallel, the same cells were centrifuged to form micromass pellets (200k cells/each) for comparison and transferred to the same 96-well plate.

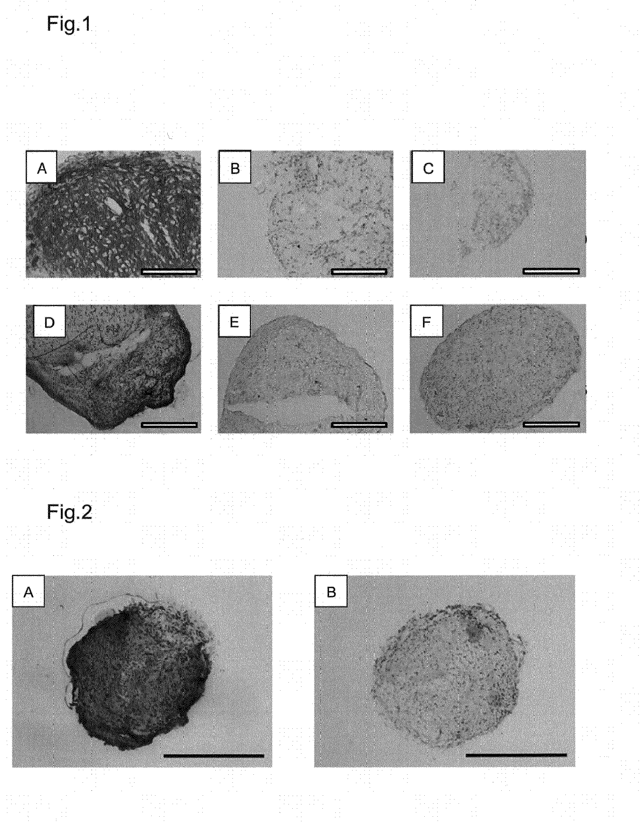

Figure 1 depicts the following possibilities:
Figure 1A - MSC-based microtissue formed via Schiff-base crosslinking using S-Chi and oxAlg with chondrogenic media.
Figure 1B - MSC-based microtissue formed via Schiff-base crosslinking using S-Chi and oxAlg with non-chondrogenic media.
Figure 1C - MSC-based microtissue formed via centrifugation (art-standard method) with chondrogenic media
Figure 1D - Adipose stem cell-based microtissue formed via Schiff-base crosslinking using S-Chi and oxAlg with chondrogenic media.
Figure 1E - Adipose stem cell-based microtissue formed via Schiff-base crosslinking using S-Chi and oxAlg with non-chondrogenic media.
Figure 1F - Adipose stem cell-based microtissue formed centrifugation (art-standard method) with chondrogenic media. Microtissues from Lonza MSCs formed via Schiff-base crosslinking using S-Chi and oxAlg demonstrated better chondrogenic induction than centrifuged pellets as indicated by alcian blue staining The intensity of the blue color shows the amount of negatively charged glycoaminoglycans produced by the cells, so in A there is much more of this molecule than in the art standard method. The practical implication is that the tissue generated looks much more like cartilage (which would also be stained dark blue)
Figure 2 shows a microtissue stained using a primary antibody against collagen 2, which is a marker of cartilage. An anti-mouse IgG1 secondary antibody and colorimetric reaction was used to visualize the localization of the protein. GAG or glycosaminoglycans are the other important part of cartilage.

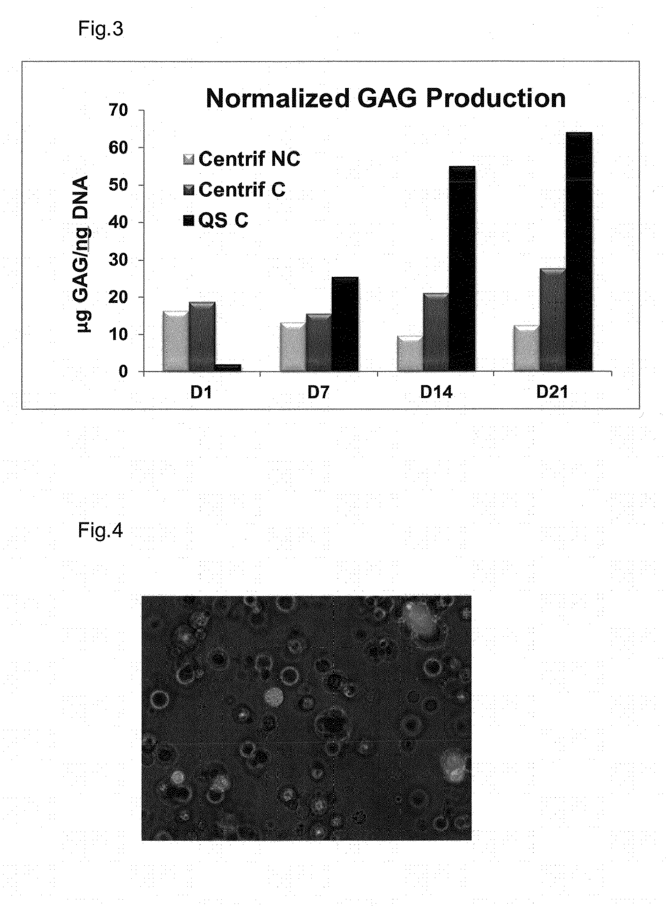

Figure 3 shows that the amount of GAG was the highest in the microtissue ("QS C"), as opposed to the centrifuged (Centrif) with chondrogenic (C) media and centrifuged with non- chondrogenic (NC) media respectively. Centrifugation is the art standard way of making microtissues.

Figure 2 shows that there is more blue staining in D compared to the art standard (however this induction was not as strong as with the bone marrow-derived MSCs).

The data indicate that crosslinked microtissues provide an immensely beneficial environment for differentiating stem cells into cartilage cells, versus the traditional technique of the art (i.e. centrifugation).

### Example 2: Use of collagen 2 promoter - GFP construct to detect individual cells which expression cartilage markers in 3D culture system.

Lentiviral chondrogenic promoter vectors comprised a transcriptional unit encoding for enhanced green fluorescent protein (EGFP) under the human collagen 2 promoter. A 6100 bp fragment of the human Col2 promoter was subcloned into pLVX-AcGFP-C1 by replacing the original CMV promoter. Subsequently, AcGFP was replaced by EGFP to create pLVX-Col(6100)-EGFP. To create pLVX-Col(-6100)-EGFP, a 1500 bp fragment and subsequently a 4600 bp fragment are excised from pKL7 and ligated into *corresponding* sites. EGFP fragment was amplified by PCR and integrated downstream the 6100 bp Col2 promoter. Correct orientation of the integrated EGFP was verified by restriction analysis.

*Transducing MSCs with Lenti-X viruses:* For transduction, the medium of the cells (seeded 12-16 hr prior transduction) was adjusted to accommodate the addition of virus and polybrene. Polybrene at a final concentration of 4 µl/ml is used to reduce the charge repulsion between the virus and the cell membrane. The lentiviral stock was diluted with medium to obtain the desired MOI and added to the cells for transduction. After 8 to 24 hr, virus-containing transduction medium was replaced with fresh growth medium. Cells are cultured and put into 3D culture to induce chondrogenic differentiation in the presence of chondrogenic medium (Dulbecco's Modified Eagle's Medium (DMEM, Gibco 41966), containing 1% Antibiotic-Antimycotic, 1% premixed IST, 100 µg/ml sodium pyruvate, 40 µg/ml L-proline, 50 µg/ml L-ascorbate-2-phosphate, 10 nM Dexamethasone, 10 ng/ml TGF-β3 (PeproTech) and 100 ng/ml BMP-2 (PeproTech))

As shown in Figure 4, in an environment which induces cartilage formation, the reporter lights up (these appear as white spots in Figure 4, green in reality). This experiment establishes the function of the collagen 2 promoter -GFP construct for detecting drugs and environmental conditions which induce cartilage matrix synthesis.

## Claims

1. A method of making a three-dimensional microtissue comprising the reaction of at least two cytocompatible polymer solutions, each polymer comprising complementary reactive groups capable of spontaneous reaction, at least one of which solutions additionally contains cells.

2. A method according to claim 1, in which the polymer is a biopolymer.

3. A method according to claim 2, in which the biopolymer is selected from the group consisting of alginate, alginate sulfate, chondroitin sulfate, dermatin sulfate, hyaluronic acid, cellulose, dextran, poly-1-lysine, chitosan, gelatin, silk and collagen.

4. A method according to claim 1, in which the polymer is s synthetic polymer.

5. A method according to claim 4, in which the polymer is selected from, or is derived from, the group consisting poly ethylene glycol, poly propylene glycol, polaxomers, polyoxazolines, polyethylenimine, poly vinyl alcohol, poly vinyl acetate, poly methyl vinyl ether-co-maleic anhydride, poly lactide, poly N-isopropylacrylamide, poly glycolic acid, poly methylmethacrylate, poly acrylamide, poly acrylic acid, polyallylamine.

6. A method according to claim 1, in which the complementary reactive groups are an aldehyde/amine pairing leading to a Schiff base linkage.

7. A method according to claim 1, in which the complementary reactive groups are participants in a Michael addition reaction.

8. A three-dimensional microtissue comprising a crosslinked composition of at least two cytocompatible polymers and cells, the crosslinking being provided by the spontaneous reaction of complementary reactive groups of two types, one of these types being present on each one of the cytocompatible polymers.

9. A screen assay, comprising a microtissue as hereinabove described, in which the cells are adapted to fluoresce on exposure to a stimulus whose detection is desired.
